# EUROPEAN PATENT APPLICATION

(11) **EP 1 038 516 A1**
(43) Date of publication of application: **27.09.2000**
(21) Application number: 00400650.8
(22) Date of filing: 09.03.2000
(51) Int. Cl.: A61K 7/06, A61K 7/42, A61K 7/48

(54) **Cosmetic composition comprising kynurenine**

(30) Priority: 16.03.1999 JP 7095299
(71) Applicant: Uchikuga, Saburoh, Yokohama-shi, Kanagawa-ken (JP)
(72) Inventor: Uchikuga, Saburoh, Yokohama-shi, Kanagawa-ken (JP)
(74) Representative: Clisci, Serge

(57) **Abstract**

The invention relates to an outdoor cosmetic comprising kynurenine (derivative) as an effective ingredient and serving for retaining skin health under exposure to ultraviolet ray, not by inhibiting tyrosinase activity but by actively utilizing tyrosinase activity and allowing the intermediate products in the course of black melanin generation to react with kynurenine (derivative) to generate safe pigments in yellowish brown to red under inhibition of the generation of black melanin.

## Description

### Detailed Description of the Invention

### Technical Field to which the Invention Belongs

The present invention relates to an outdoor cosmetic; more specifically, the invention relates to a cosmetic for outdoor use, which is preferable particularly for use at places under exposure to much ultraviolet ray, such as outdoor and which can prevent or suppress the generation of black melanin.

More specifically, the invention relates to a totally novel cosmetic being comprised with kynurenine, a kynurenine derivative or a salt thereof as an effective ingredient and being capable of retaining healthy skin at a state preferable for beauty, by effectively utilizing tyrosinase activity, with no suppression of tyrosinase activity essentially present in biological organisms and with no generation of black melanin unpreferable for beauty with tyrosinase.

### Prior Art

When sunburn occurs due to sunlight, particularly ultraviolet ray, the melanin pigment in black is formed with dermal tyrosinase, which exerts a protective function against sunlight and ultraviolet ray. However, the black melanin formed causes the deposition of the black pigment on skin, the increase of dull darkness, the decrease of transparency and the like, which are not preferable for beauty.

To suppress the generation of black melanin the agents are used: 1. glucosamine, tunicamycin and the like as tyrosinase activation inhibitors (glycosylation inhibitors) and 2. ascorbate phosphate ester magnesium salt, kojic acid, albumin and the like, as tyrosinase activity inhibitors.

In other words, these agents are used for tyrosinase activity inhibitors, inhibiting the activation of the enzyme tyrosinase playing a principal role in the generation of black melanin or suppressing tyrosinase activity; in any of the cases, tyrosinase is targeted. Nevertheless, the effects are unsatisfactory or many of them are problematic with safety concerns.

Alternatively, attempts have been made to allow glutathione and cysteine with SH group to induce the pathway to black melanin (eumelanin) to a melanin (pheomelanin) in yellowish dark brown to red to thereby suppress the generation of black melanin. However, inactivation of the SH compounds occurs due to ultraviolet ray, female hormones, copper ions and the like, so that tyrosinase is activated and rather melanin generation is promoted. Furthermore, the intermediates of the SH compounds are remarked as hazardous because the intermediates generate free radicals via ultraviolet ray (Lipids 23, 587 - 591, 1988). Hence, the effect and safety profile of the method comprising generating the yellowish dark brown melanin (pheomelanin) instead of black melanin are suspicious.

Because tyrosinase inhibition suppresses or prevents excessive formation of black melanin, with the resultant enhancement of skin whitening, alternatively, whitening cosmetics comprising tyrosinase inhibitors as effective components for the aim of whitened skin with beauty have been under way of development. The present inventor has therefore made research works previously about kynurenine (derivative). Consequently, the inventor has proposed an invention of a whitening cosmetic (Japanese Patent Laid-open No. 102018/1989) with attention focused on the tyrosinase activity-inhibiting ability of kynurenine (derivative) and the hazardous ultraviolet ray-absorbing ability thereof in sunlight. The results of the subsequent research works indicate that these actions are not enough for practical use. Thus, such whitening cosmetic has never been realized in practice.

### Problem that the Invention is to Solve

In such technically current status, the invention has been achieved. So as to completely prevent the formation of black melanin for skin whitening, two actions, namely the cutting of ultraviolet ray in the UV-A region with the highest risk for darkening skin and the inhibition of the enzyme activity of tyrosinase, should simultaneously be carried out completely. Because it is required to screen an effective component with strong such actions and use the effective component at a large quantity, serious problems in terms of side effect or safety frequently occur.

From the standpoint of skin beauty and health, the inventors have therefore acknowledged the need of the development of an absolutely new system for skin protection and the control of skin beauty and health, which is totally different from such direct countermeasures for the prevention of excessive generation of black melanin hazardous to skin as described above. The inventor has accordingly proposed herein a technical problem of the development of a new-type cosmetic conventionally unknown for outdoor use.

### Brief Description of the Drawings

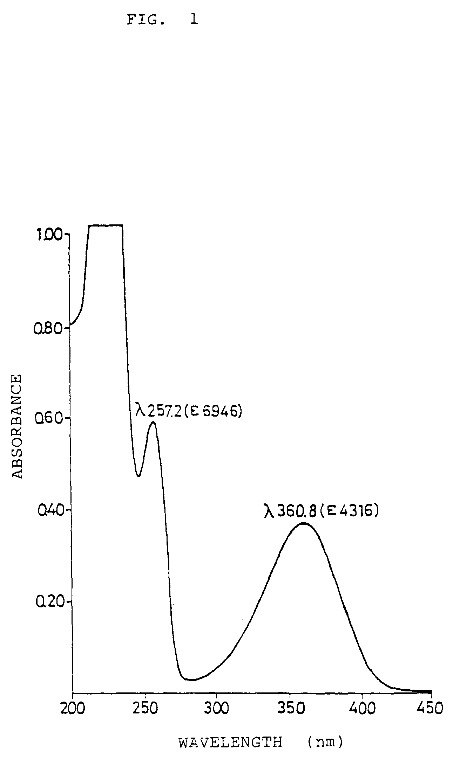

Fig. 1 shows ultraviolet absorption spectrum of DL-kynurenine sulfate salt is shown at a concentration of 8.48 × (1/10⁵) mol/L in a solvent of water.

### Means for Solving the Problem

The invention has been achieved so as to solve the problem. As results of examinations from respects of various fields, attention has again been drawn boldly toward the whitening cosmetic comprising kynurenine (derivative) as an effective ingredient, which was previously proposed by the present inventor (, et al.) but never successfully realized in practice. The whitening cosmetic of the previous application was for the purpose of whitening (not for the purpose of healthy browning at a degree of mild sunburn, namely tanning or mild skin coloring in red or the like) and was designed from the respect of the black melanin generation pathway via tyrosinase [tyrosine → dopa → dopaquinone → leucodopachrome → dopachrome → eumelanin (black melanin)] under expectation of the complete ultraviolet absorption and tyrosinase activity inhibition with kynurenine (derivative).

However, due to the subsequent research works revealing that 1. kynurenine (derivative) never has any complete absorption ability of ultraviolet ray (an unrealistically and abnormally large quantity is therefore necessarily used) and 2. kynurenine never has any ability of tyrosinase inhibition or has the ability insufficiently, consequently, the whitening effect could never be realized in practice.

Based on these reasons, the realization of the whitening cosmetic comprising as an effective ingredient kynurenine (derivative) has never successfully been attained in practice, but the research works thereafter have elucidated the concrete reasons. The detailed reasons of the unsuccessful realization of kynurenine-containing "whitening" cosmetic in practice are described below.

### 1. Absorption of UV-A:

The currently commercially available UV-A absorbent for the most common use is "Palsole A" (under trade name), as shown below.

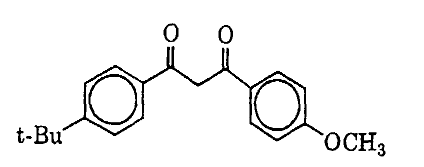

The properties of kynurenine and the derivative thereof are collectively shown below in Table 1, along with the properties of "Palsole A". As apparently shown in the table and Fig. 1, the maximum absorption lengths (λmax) of kynurenine and the derivative thereof preferably reside in 360 to 365 nm in the UV-A region (320 to 380 nm) with the highest risk of direct darkening of skin. Kynurenine has a molecular extinction coefficient (ε) functioning as a marker of the intensity of ultraviolet absorption (ε = 4,000), which is about 1/10 of the commercially available Palsole A (ε = 36,000). This indicates that so as to allow a product comprised with kynurenine and the derivative thereof to have the same efficacy as that of a product with Palsole at 1 %, the quantity of kynurenine and the derivative to be blended is required to be at 10 % corresponding to the 10-fold volume. Accordingly, such large quantity considered abnormal is required to be blended.

**Table 1**

| | Maximum absorption wavelength (λmax) | Molecular extinction coefficient (ε) |
|---|---|---|
| Inventive substance | 360 to 365 nm | 4,000 |
| Palsole A | 355 nm | 35,000 |

### 2. With respect to absorption in other ultraviolet regions

In addition to the UV-A region, kynurenine and the derivative thereof have the maximum absorption wavelength at 253 nm with the molecular extinction coefficient of 6,700. In other words, kynurenine and the derivative thereof exert absorption in the UV-C region (10 to 290 nm). The ultraviolet ray in the UV-C region is generally absorbed in the ozone layer and therefore never reaches ground surface, so the absorption in the region is not meaningful as cosmetics.

Kynurenine and the derivative thereof never exert absorption in the UV-B region (290 to 320 nm) in the erythema curve region with the highest risk in sunlight. Thus, an effect on the prevention of erythema against ultraviolet ray in the UV-B region cannot be expected.

### 3. Tyrosinase activity-inhibiting effect

It was expectantly assumed that kynurenine and the derivative thereof inhibited tyrosinase and thereby suppressed the generation of black melanin so that kynurenine and the derivative thereof could be utilized as an ingredient for preventing spot and freckle and the deposition of pigments. However, the results of detailed examination indicated that kynurenine and the derivative thereof never had any tyrosinase-inhibiting ability.

In the previous application, propositions were made to utilize kynurenine (derivative) as an ingredient for preventing the deposition of black melanin by allowing kynurenine to exert the ultraviolet absorption ability and the tyrosinase activity-inhibiting ability. As described in the reasons 1 to 3, however, the ultraviolet absorption ability was not enough to inhibit tyrosinase activity. Ultimately, no success was attained in obtaining any "whitening" cosmetic for the aim of whitening and skin beauty.

Although any "whitening" cosmetic comprising kynurenine (derivative) as an effective ingredient was unsuccessful as described above, an absolutely novel standpoint was reached from a new respect of the prevention of the generation of black melanin alone for the aim of the control of skin beauty and health as described above, without attention singly focused on "whitening" for serving for whitening skin, such that even if cosmetics specific for outdoor use under exposure to sufficient sunlight (outdoor cosmetic) involved healthy mild sunburn never caused by black melanin but caused by the generation of pigments in yellowish brown to red, the cosmetics could sufficiently retain the beauty and health of skin.

While attention was again drawn on the kynurenine (derivative) unusable as "whitening" cosmetics, examinations were positively carried out about pigments except black melanin pigment targeted in the "whitening" cosmetic of the previous application. Quite unexpectedly, a new finding was consequently recovered that 3-hydroxykynurenine reacted with dopaquinone generated via tyrosinase action, to generate safe pigments in yellowish brown to red, unlike black melanin. Then, a novel idea was hinted that the presence of a large quantity of kynurenine might reduce the quantity of dopaquinone or might eliminate dopaquinone, so that the generation of black melanin might be inhibited or suppressed. Simultaneously, an idea was freshly hinted as to the use of an outdoor cosmetic or a cosmetic for rather healthy mild sunburn, so-called tanning, because the generation of pigments in yellowish brown to red is no problem for the beauty and health of skin.

So as to confirm the novel ideas, then, the following test examples were carried out. In other words, the relations of dopa, tyrosine, and tyrosinase with (3-hydroxy)kynurenine were tested on the basis of the following samples. The following results were individually recovered from the tests.

### Test Example 1

5 units of tyrosinase, 2.5 µmol of dopa or tyrosine, and 1 mg of kynurenine sulfate salt or 3-hydroxykynurenine dihydrate were dissolved in 0.1M phosphate buffer, pH 7.0 to a final volume of 5 ml and the resulting solution was agitated at ambient temperature for 3.5 hours; thereafter, the disappearance of kynurenine or 3-hydroxykynurenine was assayed at 355 nm, which corresponds to the λmax of kynurenine and 3-hydroxykynurenine in UV-A. The contents of the individual tests are shown below in Table 2. The results are shown below in Table 3.

**Table 2**

| Test No. | Tyrosinase | Dopa/tyrosine | Kynurenine | 3-Hydroxykynurenine | Air |
|---|---|---|---|---|---|
| 1 | + | + | + | - | + |
| 2 | + | + | - | + | + |
| 3 | thermal treatment | + | - | + | + |
| 4 | + | - | - | + | + |
| 5 | + | + | + | - | - |

**Table 3**

| Test No. | Results |
|---|---|
| 1. | kynurenine disappeared; pigment generated; λmax = 450 nm observed. |
| 2. | 3-hydroxykynurenine disappeared; pigment generated; λmax = 455 nm observed. |
| 3. | no disappearance of 3-hydroxykynurenine observed. |
| 4. | no disappearance of 3-hydroxykynurenine observed. |
| 5. | no disappearance of kynurenine observed. |

It was confirmed from the above results that no disappearance of 3-hydroxykynurenine/kynurenine or no generation of any dark brown pigment was observed in the following cases. In other words, it was verified that pigments with λmax at 450 to 455 nm were generated by the reaction of kynurenine/3-hydroxykynurenine through the oxidation with tyrosinase.
1. A case that tyrosinase is inactivated by thermal treatment.
2. A case that tyrosine/dopa as tyrosinase substrate is absent.
3. A case of no presence of oxygen.

The results of further examination verify that one of the generated pigments in yellowish brown to red is xanthommatin with the following formula II. Xanthommatin is the brown pigment of eye and a pigment essentially present in biological organisms, which is therefore a safe substance.

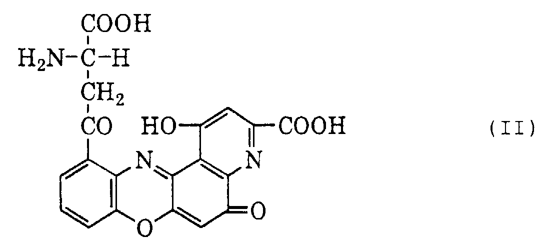

The aforementioned properties of kynurenine and the derivative thereof, the reaction with dopaquinone, the generation of pigments in yellowish brown to red and the like, were generally examined. Consequently, it was elucidated that various troubles could be prevented by the mild UV-A absorbing action in the UV-A region whereby the overdose of sunlight could be avoided, and additionally that novel pigments were generated from kynurenine and the derivative thereof and tyrosine and substances relating thereto with tyrosinase action under the influence of an appropriate dose of ultraviolet ray never absorbed. Thus, the invention has been achieved.

In other words, it was confirmed that pigments in yellowish brown to red were newly generated through the reaction between kynurenine and the derivative thereof and tyrosine and substances relating thereto, by never inhibiting but utilizing the enzyme reaction of tyrosinase activated under the influence of an appropriate dose of ultraviolet ray never absorbed, under avoidance of the overdose of sunlight through the mild UV-A absorbing action of kynurenine and the derivative thereof. It is verified that by using the inventive cosmetic comprised with kynurenine and the derivative thereof, healthy and beautiful skin with less dull darkness and with transparency can be retained under exposure to ultraviolet ray such as outdoor. Thus, the invention has been attained.

More specifically, the invention provides an outdoor cosmetic preferable for use at outdoor under exposure to intense ultraviolet ray, in particular, which contains at least one of kynurenine represented by the following general formula I, the derivative thereof and a salt thereof as an effective ingredient and in which tyrosinase action is actively utilized in a fashion to generate safe non-melanin pigments in yellowish brown to red during the course of tyrosine oxidation and thereby prevent excessive generation of hazardous black melanin pigment, the fashion being completely opposite to conventional "whitening" cosmetics under inhibition of tyrosinase activity to prevent the generation of black melanin; or the invention provides a cosmetic for tanning, serving for making healthy and beautiful skin in brown thereby enabling the control of the beauty and health of skin owing to the generation of the non-black melanin pigments in yellowish brown to red. 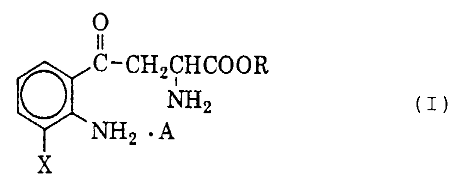 (in the formula, X represents hydrogen or hydroxyl group or forms a glycoside with hydroxyl group and glucose; R represents hydrogen or a lower alkyl group; and A is free or represents a mineral acid or acid mucopolysaccharides; with sucrose, Zn or Cu, the compound of the formula forms a complex.)

The relation between the xanthommatin generation mechanism and the black melanin generation mechanism in accordance with the invention is deduced as follows at present.

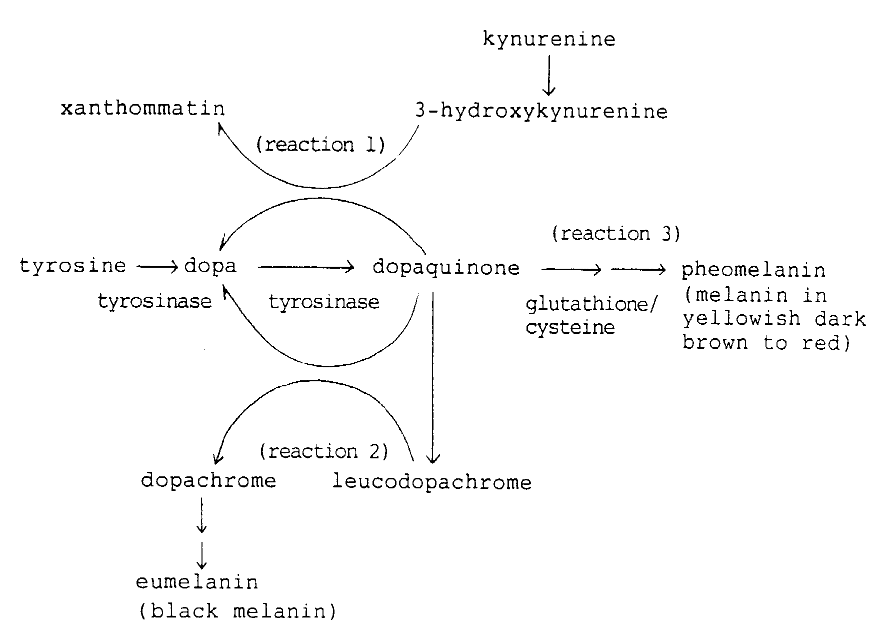

In other words, 3-hydroxykynurenine/kynurenine react with phenyl orthoquinone, namely dopaquinone and indole orthoquinone, namely dopachrome to independently generate dihydroxyphenol, namely dopa and dihydroxyindole, namely leucodopachrome, respectively. This indicates that the inverse flow of tyrosine → dopa → dopaquinone → leucodopachrome → dopachrome → eumelanin (black melanin) (reaction 2) can prevent the generation of black melanin. Additionally, dopaquinone oxidizes 3-hydroxykynurenine to generate a brown pigment xanthommatin (reaction 1), while together with glutathione/cysteine, dopaquinone generates pheomelanin as the melanin in yellowish brown to red (reaction 3). But the reaction is problematic in light of the effect and safety profile, as described above.

At outdoor, black melanin pigment is formed through tyrosinase action in sunlight, particularly ultraviolet ray. However, black melanin formed causes the deposition of the black pigment on skin, the increase of dull darkness, the reduction of transparency and the like, unpreferably for beauty. In accordance with the invention, the action of the enzyme tyrosinase essentially present is utilized to generate a brown pigment with no generation of black melanin. Thus, a brown healthy pigment preferable for beauty can be generated with no increase of dull darkness or no reduction of transparency, so that the health and beauty of skin can sufficiently be retained by such mild method, without essential whitening of skin. Such so-called indirect method for indirectly preventing the generation of black melanin is a novel method totally different from conventional direct methods for preventing the generation of black melanin comprising inhibiting tyrosinase activity and the like, and serves as an absolutely new system for preventing black melanin generation, which is first accomplished herein. Additionally, the cosmetic can be used for a novel use as a tanning cosmetic.

Kynurenine as one of the effective ingredient of the cosmetic of the invention can be relatively readily available from tryptophan according to the method described in Methods in Enzymol., III, 620 (1957); 3-hydroxykynurenine can be relatively readily available from kynurenine according to the method described in Biochem. Prep., Vol. 7, 63; and 3-hydroxykynurenine-o-glucoside can be relatively readily available from animal eyes according to known methods, for example the method described in J. Biochem., Vol. 89, 103 (1981). Additionally, salts of kynurenine or 3-hydroxykynurenine with acid mucopolysaccharides can be readily recovered when both of the two components are simply mixed together. Therefore, the two components are satisfactorily mixed together during the course of the process of producing the product. Rodent hair containing a high content of kynurenine can be utilized; otherwise, the decomposition extracts thereof can be utilized as well. The complex of kynurenine (derivative) with sucrose, Zn and Cu can be recovered, readily, according to the method in Z. physiol. Com. Bd. 278. 27. (1943) Bd. 298. 169 (1954).

In the compound represented by the general formula I, the lower alkyl group includes for example linear or branched alkyl groups with 10 or less carbon atoms, such as methyl, ethyl, propyl, butyl, pentyl, hexyl, isopropyl and isobutyl groups. The mineral acid includes hydrochloric acid, sulfuric acid, nitric acid, phosphoric acid and others.

The acid mucopolysaccharides widely include acid mucopolysaccharides comprising N-acetylglucosamine, glucosamine and the like, and include for example hyaluronic acid, heparin, hyaluronosulfuric acid, chondroitin sulfuric acid, and phosphomucopolysaccharides.

Additionally, the salt of the kynurenine (derivative) widely includes mineral acid salts such as hydrochloride salt, sulfate salt and others; and organic acid salts such as citrate salt, succinate salt and others. Furthermore, the kynurenine (derivative) is of a property forming a complex with sucrose, Zn and Cu.

The kynurenine represented by the general formula I and substances relating thereto are metabolic intermediates of tryptophan and are essentially present in biological organisms; and the kynurenine and substances relating thereto are contained at particularly large quantities in the eyes of human new born babies and infants but are decreased over aging [J. Biochem., Vol. 89, 103 (1981)], and have therefore no safety problem. The results of Ames test (test with 5 bacterial strains) indicate that no mutagenicity was observed in any of them.

The generated brown pigment is present in the eye, skin and wing of Arthropoda and Mollusca. The brown pigment is also contained in human eye. Because the brown pigment is widely distributed in animals in such manner, the generated pigment is greatly safe. The brown pigment (xanthommatin) was chemically synthesized according to the method in Angew. Chem., 69, Jahrg. 16 (1957) and subjected to Ames test. No mutagenicity was observed, which indicates that the brown pigment is totally safe.

As the inventive substance and the brown pigment as a generated product are quite safe with no problem as described above, the content thereof for use is not necessarily limited. But the content is satisfactorily 0.01 % to 50 %, preferably 0.05 % to 8 %, more preferably 0.08 % to 3 %.

If necessary, the inventive cosmetic can be blended with an oily component, a moisturizing agent, a surfactant, a mildew proofing agent, fragrance and the like, to prepare skin care products and hair care products according to conventional methods. So as to prepare the inventive cosmetic, more specifically, routine methods for preparing cosmetics and external agents are adopted, with no limitation to bases for use. Therefore, the inventive cosmetic can be blended appropriately with fats and fatty oils, moisturizing agents, pigments, dyes, surfactants, antioxidants, UV-B ultraviolet absorbing agents, mildew proofing agents, water-soluble polymers and the like, which are generally blended in pharmaceutical products, quasi-drugs, cosmetics and the like.

The inventive compound is of optical isomerism and is generally in the L form. However, the D form and DL form are also subjected to the purpose of the use.

The effect of the cosmetic of the invention was confirmed in the following test example.

### Test Example 2

Two types of creams for use at test, namely base cream for use as control and kynurenine-comprising cream, were prepared according to the following formulations.

| | Base cream | Cream of 5 % kynurenine sulfate salt |
|---|---|---|
| 1. Glycerin monostearate | 1.2 | 1.2 |
| 2. Polyoxyethylene behenyl ether | 1.8 | 1.8 |
| 3. Bee wax | 3.0 | 3.0 |
| 4. Batyl alcohol monostearate | 2.0 | 2.0 |
| 5. Batyl alcohol | 1.0 | 1.0 |
| 6. Cetyl isooctanate | 18.0 | 18.0 |
| 7. 1,3-Butylene glycol | 5.0 | 5.0 |
| 8. Methylparabene | 0.1 | 0.1 |
| 9. Propylparabene | 0.1 | 0.1 |
| 10. Sodium dehydroacetate | 0.1 | 0.1 |
| 11. Kynurenine sulfate salt | 0 | 5.0 |
| 12. | Add water to a final total volume of 100. | |

Using the base cream and the 5 % kynurenine-comprising cream, 20 female volunteers were coated on three pairs of left and right regions on their backs at 2.0 mg/cm². Three days after sun bathing on sea shore in summer sunshine for a total of 0.5 hour, the female volunteers judged the effects from the respects of "tanning effect", "effect on the reduction of dull darkness", "transparency increase", and "general good feeling". The results are as shown in the following table.

**Table 4**

| Results of panel tests | | | | |
|---|---|---|---|---|
| Degree of effect | Number of persons enrolled in the assessment | | | |
| | tanning effect | effect on reduction of dull darkness | effect on transparency increase | general effect |
| Prominent effect | 18 | 18 | 15 | 17 |
| Considerable effect | 1 | 1 | 2 | 2 |
| Slight effect | 1 | 3 | 3 | 1 |
| No effect | 0 | 0 | 0 | 0 |

The examples of the invention are now described below, but the invention is not limited to only these examples.

| Example 1: Production of skin cream | |
|---|---|
| 1. Kynurenine sulfate salt | 5.0 |
| 2. Stearic acid | 10.0 |
| 3. Cetyl alcohol | 1.0 |
| 4. Glyceryl monomyristate | 5.0 |
| 5. Isopropyl myristate | 7.0 |
| 6. Oleyl alcohol | 4.0 |
| 7. DEA cetyl phosphate | 3.0 |
| 8. Desalted water | 63.5 to 62.5 |
| 9. Propylene glycol | 6.0 |
| 10. Fragrance and preservative | appropriate amounts |

Under agitation, the components 1 to 6 are heated up to 85 °C; the component 7 is added to the resulting mixture while the mixture is retained at the temperature. When the mixture turns homogenous, the components 8 to 9 are added to the mixture, which is continuously agitated until the temperature reaches 40 °C. After homogenization, the component 10 is added to the mixture, which is then cooled to ambient temperature.

| Example 2: Production of hair care lotion | |
|---|---|
| 1. 3-Hydroxykynurenine dihydrate | 3.0 |
| 2. Cetyl alcohol | 2.0 |
| 3. Glyceryl monomyristate | 4.0 |
| 4. Isopropyl myristate | 5.0 |
| 5. DEA-cetyl phosphate | 3.0 |
| 6. Desalted water | 74.5 to 75.5 |
| 7. Propylene glycol | 3.0 |
| 8. Urea | 5.0 |
| 9. Fragrance and preservative | slight amounts |

Under agitation, the components 1 to 4 are heated up to 85 °C; the component 5 is added to the resulting mixture while the mixture is retained at the temperature. When the mixture turns homogenous, the components 6 to 8 are added to the mixture, which is continuously agitated until the temperature reaches 40 °C. After homogenization, the component 9 is added to the mixture, which is then cooled to ambient temperature.

### Advantages of the Invention

The inventive cosmetic represented by the general formula I is a substance widely distributed in the animal kingdom, which generates the brown pigment (of general formula II) present in biological organisms with no inhibition of tyrosinase activity, instead of black melanin essentially generated through the reaction system of tyrosinase, dopa and dopaquinone, exerts the tanning effect, the effect on the reduction of dull darkness, and the effect on the increase of transparency and further exerts the effect of realizing sunburn skin healthy and preferable from general views.

In other words, with attention again focused on the fact that even if other pigments except black melanin are generated, the pigments are not relatively problematic for the health and beauty of skin, provided that the pigments are not at excessively high concentrations and never exert hazardous actions, the invention has been attained (not by inhibiting tyrosinase activity as observed conventionally but by actively utilizing tyrosinase action, on contrast) for the purpose of developing a cosmetic suitable for outdoor life, namely a new-type cosmetic as an outdoor cosmetic retaining skin health while approving the generation of non-hazardous pigments for the beauty and health of skin, unlike black melanin to thereby inhibit excessive generation of black melanin, absolutely unlike whitening cosmetics for the aim of complete whitening, on the basis of an innovative idea converted from conventional ideas, such that a cosmetic utilizing so-called tanning meaning mild dermal sunburn to prepare healthy brown skin preferable for beauty or the generation of pigments in yellowish brown to red due to appropriate outdoor sunburn would be more realistic from the standpoint of skin beauty. Such novel technical problem has first been overcome with success by using kynurenine (derivative).

## Claims

1. An outdoor cosmetic comprising at least one selected from kynurenine represented by the following general formula I, a kynurenine derivative, and a salt thereof as an effective ingredient and generating non-black melanin pigments in yellowish brown to red with no generation of black melanin by utilizing the action of the enzyme tyrosinase with no total absorption of ultraviolet ray in the UV-A region: 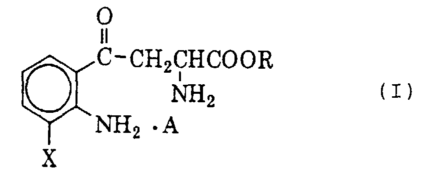 (in the formula, X represents hydrogen or hydroxyl group or forms a glycoside with hydroxyl group and glucose; R represents hydrogen or a lower alkyl group; and A is free or represents a mineral acid or acid mucopolysaccharides; with sucrose, Zn or Cu, the compound of the formula forms a complex.)

2. An outdoor cosmetic according to claim 1, wherein the content of the effective ingredient is not a quantity absorbing totally ultraviolet ray in the UV-A region, to prevent the overdose of sunlight through the mild ultraviolet absorbing action in the UV-A region and wherein the enzyme reaction of the enzyme tyrosinase activated with the action of an appropriate dose of ultraviolet ray never absorbed is not inhibited but utilized to generate non-black melanin pigments in yellowish brown to red.

3. An outdoor cosmetic according to claim 1 or 2, wherein the non-black melanin pigments in yellowish brown to red are xanthommatins.

4. An outdoor cosmetic according to any one of claims 1 to 3, wherein the outdoor cosmetic is for tanning use.
